# EUROPÄISCHE PATENTSCHRIFT

(11) **EP 0 487 073 B1**
(45) Veröffentlichungstag und Bekanntmachung des Hinweises auf die Patenterteilung: **03.09.1997**
(21) Anmeldenummer: 91119834.9
(22) Anmeldetag: 19.11.1991
(51) Int. Cl.: A61B 3/02, A61B 3/032, A61B 3/08

(54) **Vorrichtung zur Untersuchung des Sehvermögens**
Device for examining visual acuity
Dispositif pour l'examen de l'acuité visuelle

(30) Priorität: 20.11.1990 DE 4036964
(43) Veröffentlichungstag der Anmeldung: 27.05.1992
(73) Patentinhaber: G. RODENSTOCK INSTRUMENTE GMBH, 85521 Ottobrunn (DE)
(72) Erfinder: Wilms, Karl-Heinz, W-8089 Emmering (DE)
(74) Vertreter: Münich, Wilhelm, Dr.

(56) Entgegenhaltungen:
- WO-A-88/03396
- WO-A-91/00050
- US-A- 4 300 818
- US-A- 4 726 673

## Beschreibung

Die Erfindung bezieht sich auf eine Vorrichtung zur Untersuchung des Sehvermögens mit einem binokularen Strahlengang, durch den die untersuchte Person Sehzeichen oder dergleichen betrachtet, gemäß dem Oberbegriff des Patentauspruchs 1 .

Beispiele für derartige Vorrichtungen zur Untersuchung des Sehvermögens mit einem binokularen Strahlengang sind Sehtestgeräte oder sogenannte Brillenbestimmungsgeräte.

Bei einem Sehtestgerät - wie es beispielsweise aus dem deutschen Gebrauchsmuster 19 63 161 bekannt ist - blickt die untersuchte Person über ein Linsen- und/oder Spiegelsystem, das einen definierten Teilstrahlengang für jedes Auge erzeugt und das darüberhinaus spezielle Bedingungen, wie Fernsicht- oder Nahsicht-Bedingungen herstellen kann, auf einen Sehzeichenträger, der Sehzeichen, wie Optotypen, verschieden große Buchstaben oder Ziffern usw. darbietet.

Zum Umschalten zwischen binokularer und monokularer Betrachtung ist bei den bekannten Sehtestgeräten zumeist eine Schwenkblende vorgesehen, die entweder in den einen oder den anderen Strahlengang einschwenkbar oder vollständig aus beiden Strahlengängen herausschwenkbar ist.

Hiervon zu unterscheiden sind Sehtestgeräte, wie sie beispielsweise aus der DE 31 33 608 C2 bekannt sind. Derartige Sehtestgeräte anderer Gattung als im Oberbegriff des Patentanspruchs 1 vorausgesetzt bestehen im wesentlichen nur aus einem Sehzeichenträger, auf den die zu untersuchende Person direkt, d.h. ohne zwischengeschaltetes Linsen- und/oder Spiegelsystem blickt. Diese Sehtestgerät haben zwar den Vorteil, daß sie vergleichsweise einfach aufgebaut sind; nachteilig ist jedoch, daß kein definierter mono- oder binokularer Strahlengang vorhanden ist.

Ein Gerät zur Darbietung von Sehzeichen unter Verwendung einer Brille ist aus der US-A-4 726 673 bekannt.

In die Brillenfassung sind LCD-Verschlüsse eingesetzt, die den jeweiligen Strahlengang öffnen bzw. schließen.

Bei einem sogenannten Brillenbestimmungsgerät sind in jeden Teil-Strahlengang Testlinsen mit einer bestimmten optischen Wirkung einbringbar. Hierzu wird beispielsweise auf die US-PS 4 606 624 verweisen. Die untersuchte Person blickt mono- oder binokular durch das Brillenbestimmungsgerät auf einen Sehzeichenträger und teilt dem Augenarzt bzw. dem Augenoptiker mit, bei welcher Wirkung sie Sehzeichen bestimmter Größe am besten erkennen kann. Die derzeit gebräuchlichen Brillenbestimmungsgeräte weisen dabei eine Reihe von in Richtung der optischen Achse hintereinander angeordneten Scheiben (Recoss-Scheiben) auf, von denen jede eine Mehrzahl von Testlinsen trägt. Die Testlinsen können so in den Strahlengang eingebracht werden, daß sich in dem jeweiligen Teil-Strahlengang eine bestimmte optische Wirkung ergibt, wobei die optischen Wirkungen in den beiden Teilstrahlengängen unabhängig voneinander mit einer feinen Abstufung einstellbar sind. Zusätzlich sind auf den Recoss-Scheiben, die die Testlinsen tragen, an verschiedenen Plätzen Blenden vorgesehen, die es ermöglichen, zur Realisierung eines monokularen Einblicks den jeweiligen Teil-Strahlengang zu schließen, wobei zum Schließen des Strahlengangs die Recoss-Scheibe "nur über wenige Plätze" gedreht werden muß.

Die Verwendung derartiger mechanischer Blenden hat nun eine Reihe von Nachteilen:

Das Vorsehen einer Reihe von Blenden bzw. von Verschlußelementen auf einer Recoss-Scheibe verringert die Zahl der auf der Recoss-Scheibe unterbringbaren Testlinsen. Andererseits erfordern von den Recoss-Scheiben getrennte Schwenkblenden oder dgl. einen vergleichsweise großen mechanischen Aufwand.

Vor allem aber ist mit mechanischen Blenden zumindest dann, wenn der mechanische Aufwand nicht zu hoch sein soll, kein schnelles Umschalten zwischen den beiden Teilstrahlengängen möglich.

Der Erfindung liegt die Aufgabe zugrunde, eine Vorrichtung zur Untersuchung des Sehvermögens mit einem binokularen Strahlengang, durch den die untersuchte Person Sehzeichen oder dergleichen betrachtet, derart weiterzubilden, daß bei vergleichsweise geringem Aufwand nicht nur schnell zwischen binokularem und monokularem Strahlengang bzw. zwischen den einzelnen Teilstrahlengängen umgeschaltet werden kann, sondern auch eine wesentlich flexiblere und variantenreichere Untersuchungsführung als bei in bekannter Weise angeordneten LCD-Verschlüssen möglich ist.

Eine erfindungsgemäße Lösung dieser Aufgabe ist im Anspruch 1 angegeben.

Erfindungsgemäß ist in jedem Teilstrahlengang des binokularen Strahlengangs ein LCD-Verschluß vorgesehen, der durch eine Steuereinheit ansteuerbar ist. Die Steuereinheit steuert die LCD-Verschlüsse derart, daß ein oder beide Teilstrahlengänge synchronisiert kontinuierlich geöffnet und geschlossen werden. Weiterhin werden die LCD-Verschlüsse selektiv derart angesteuert, daß sie den Strahlengang in bestimmter, vorgebbarer Weise in Form eines Musters abschwächen.

LCD-Verschlüsse, die im Gegensatz zu der in der DE 31 33 608 C2 beschriebenen Vorrichtung nicht im Bereich des Sehzeichenträgers, sondern im Beobachtungs-Strahlengang und im Bereich des optischen Systems mit optischer Wirkung jedes Teilstrahlengangs vorgesehen sind, ermöglichen - wie im folgenden noch näher ausgeführt wird - eine wesentlich flexiblere und variantenreichere Untersuchungsführung als in bekannter Weise angeordnete LCD-Verschlüsse. Darüberhinaus haben sie auch gegenüber mechanischen Blenden bzw. Verschlüssen eine Reihe von Vorteilen:

Die vorgenannten Eigenschaften von LCD-Verschlüssen ermöglichen es darüberhinaus, daß die Steuereinheit jeden LCD-Verschluß derart ansteuert, daß er den jeweiligen Teilstrahlengang bzw. das Muster kontinuierlich öffnet bzw. schließt. Damit kann beispielsweise eine für die untersuchte Person nicht spürbare Umschaltung von binokularem auf monokularen Strahlengang bzw. eine Umschaltung zwischen den Teilstrahlengängen realisiert werden. Darüberhinaus ist die Ausbildung bei der Prüfung des Dämmerungssehvermögens hilfreich.

Das Umschalten zwischen lichtdurchlässig/lichtundurchlässig erfolgt bei einem LCD-Verschluß zumindest dann wesentlich schneller als bei einem mechanischen Verschluß, wenn versucht wird, den Aufwand für den mechanischen Verschluß vergleichsweise gering zu halten. Anders ausgedrückt sind LCD-Verschlüsse wesentlich kostengünstiger als vergleichbar schnelle mechanische Verschlüsse bzw. Blenden.

Weiterhin kann durch die erfindungsgemäße Ausbildung sehr viel schneller als mit mechanischen Verschlüssen von einem monokularen Strahlengang auf den anderen monokularen Strahlengang bzw. zurück auf einen binokularen Strahlengang "geschaltet" werden.

Darüberhinaus ist der Aufwand bei der Ansteuerung der Verschlüsse deutlich geringer. Insbesondere ist es wesentlich einfacher, zwei LCD-Verschlüsse als zwei mechanische Verschlüsse zu synchronisieren, so daß auch problemlos eine abwechselnde Darstellung der Sehzeichen für das linke Auge und das rechte Auge in kurzen Zeitabständen erfolgen kann.

Vor allem aber ist bei einem LCD-Verschluß eine selektive Ansteuerung möglich. Hiermit ist gemeint, daß der LCD-Verschluß nicht nur zwischen lichtdurchlässig und "vollständig lichtundurchlässig" umgeschaltet werden kann, sondern daß es auch möglich ist, den Strahlengang in bestimmter, einfach vorgebbarer Weise abzuschwächen.

Damit kann mit einer ansonsten herkömmlich aufgebauten Vorrichtung zusätzlich zur Untersuchung des Sehvermögens eine Überprüfung des Dämmerungs-Sehvermögens erfolgen.

Da die LCD-Verschlüsse nicht im Bereich des Sehzeichenträgers, sondern im Bereich des optischen Systems des Untersuchungsgeräts angeordnet sind, spielen Verzeichnungen, die bei LCD-Verschlüssen auftreten können, eine wesentliche geringere Rolle als bei LCD-Verschlüssen, die unmittelbar bei der Betrachtungsebene angeordnet sind.

Weiterbildungen der Erfindung sind Gegenstand der Unteransprüche.

Das Muster kann nach Anspruch 2 beispielsweise ein Zentrierkreuz oder eine Lochblende sein; auch kann der LCD-Verschluß die Zusatzfunktion einer Zwischenblende (Anspruch 3) wahrnehmen.

Der erfindungsgemäß vorgesehene LCD-Verschluß kann dabei in herkömmlich aufgebaute Sehtestgeräte bzw.

Brillenbestimmungsgeräte problemlos auch dann integriert werden, wenn diese Geräte bislang noch keinen mechanischen Verschluß aufgewiesen haben:

Bei einem Brillenbestimmungsgerät ist es beispielsweise möglich, den LCD-Verschluß anstelle herkömmlicher Staubschutzgläser vor oder hinter den Testlinsen anzuordnen (Anspruch 4).

Bei einem Sehtestgerät, wie es beispielsweise in dem deutschen Gebrauchsmuster 19 63 161 beschrieben ist, kann der LCD-Verschluß anstelle der herkömmlichen Schwenkblende zwischen dem Linsensystem angeordnet werden, das die Bedingungen für Fernsicht herstellt (Anspruch 5).

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1a und 1b: ein Brillenbestimmungsgerät in teilweise aufgebrochener Aufsicht bzw. im Querschnitt bei I-I in Figur 1a, und
- Fig. 2: ein Sehtestgerät.

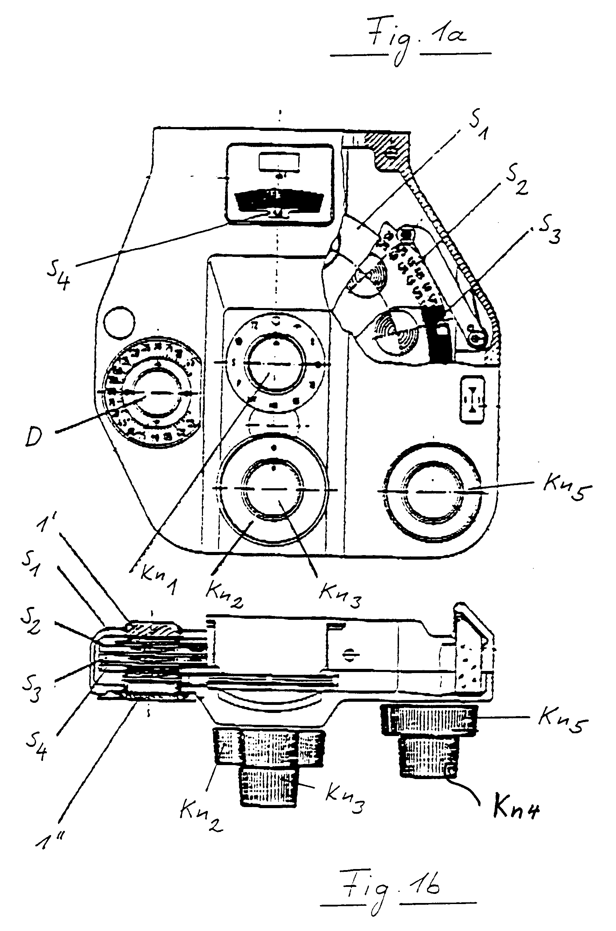

Figur 1 zeigt einen Teil eines an sich bekannten Brillenbestimmungsgerätes, und zwar den Teil, durch dessen Durchblicköffnung D (s. Fig. 1a) die untersuchte Person mit dem linken Auge blickt (in Figur 1a ist eine Aufsicht auf die der Untersuchungsperson zugewandte Seite dargestellt). Das Brillenbestimmungsgerät weist drei Trägerscheiben (auch als Recoss-Scheiben bezeichnet) S₁, S₂, S₃ und S₄ für Testgläser, Filter etc. auf.

In der dem Auge der untersuchten Person am nächsten liegenden Trägerscheibe S₁ befinden sich die mit einem Knopf Kn₁ einstellbaren Sonderteste. Die Trägerscheibe S₂ trägt die in 3 dpt abgestuften Linsen, während die Trägerscheibe S₃ die diese Intervalle überbrückenden Gläser in 0,25 dpt Abstufung trägt. Die zugehörigen Bedienungsknöpfe sind Kn₂ und Kn₃. In der Trägerscheibe S₄ sind als Vorschaltlinsen zur Trägerscheibe S₂ Zylinderlinsen anzusehen. Sie werden am Knopf Kn₄ eingeschaltet und durch den Drehknopf Kn₅ der Achse nach eingestellt.

Soweit wie vorstehend beschrieben ist das in den Fig. 1a und 1b dargestellte Brillenbestimmungsgerät bekannt und wird von der G. Rodenstock Instrumente GmbH unter der Bezeichnung "Phoromat" hergestellt und vertrieben.

Erfindungsgemäß sind entweder in die vordere oder hintere Abdeckscheibe der Durchblicköffnung beider Strahlengänge jeweils ein LCD-Verschluß 1' bzw. 1'' integriert, den eine nicht gezeigte Steuereinheit, die in an sich bekannter Weise aufgebaut ist, derart ansteuert, daß ein oder beide Teilstrahlengänge synchronisiert geöffnet und geschlossen werden.

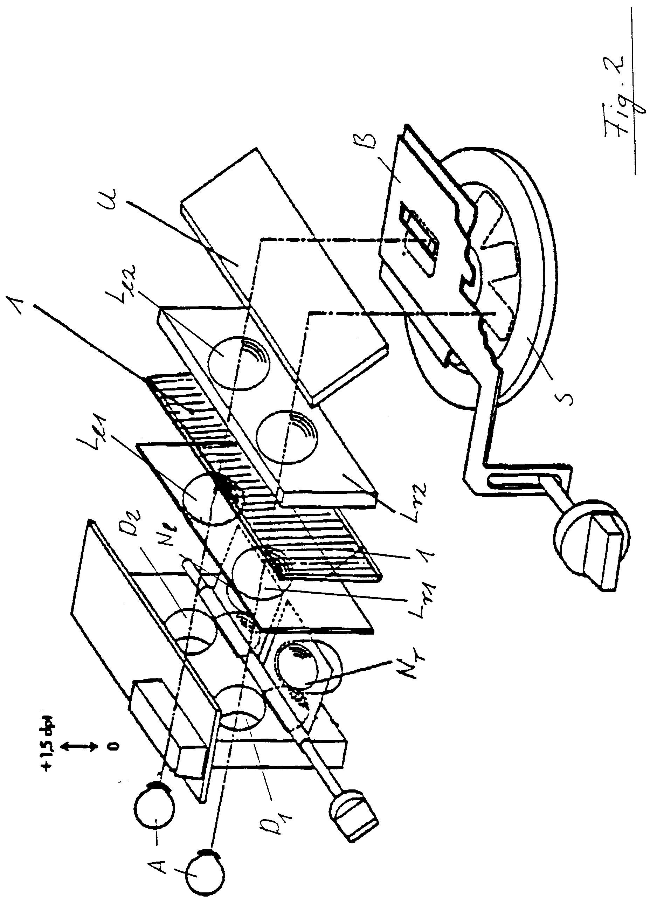

Figur 2 zeigt perspektivisch den binokularen Strahlengang eines Sehtestgeräts. Dabei sind die beiden Augen der untersuchten Person schematisch mit A bezeichnet. Das Sehtestgerät weist Einblicköffnungen D₁ und D₂ auf.

Im Strahlengang befindet sich ein aus jeweils zwei Linsen bestehendes erstes Linsensystem Lₗ₁..Lᵣ₂, das Fernsicht-Bedingungen für die untersuchte Person herstellt. Der Strahlengang wird durch einen Umlenkspiegel U auf eine Testscheibe S abgelenkt, auf der sich eine Reihe von Mustern befinden, von denen für die untersuchte Person jeweils nur eines durch eine Blende B sichtbar ist. Weiterhin ist ein einschwenkbares Linsensystem Nₗ bzw. Nᵣ vorgesehen, das Nahsicht-Bedingungen herstellt.

Soweit wie vorstehend beschrieben ist das Sehtestgerät bekannt und wird ebenfalls von der bereits genannten G. Rodenstock Instrumente GmbH hergestellt und vertrieben.

Erfindungsgemäß sind zwischen den beiden Linsen Lₗ₁ und Lₗ₂ und Lᵣ₁ bzw. Lᵣ₂ des Linsensystems, das Fernsicht-Bedingungen herstellt, LCD-Verschlüsse 1 vorgesehen.

Die LCD-Verschlüsse, die erfindungsgemäß in den in den Figuren 1 und 2 dargestellten Vorrichtungen zur Untersuchung des Sehvermögens integriert sind, sind derart selektiv ansteuerbar, daß sie in Form eines Muster den Teilstrahlengang öffnen bzw. schließen. Selbstverständlich können die Verschlüsse aber auch einen oder beide Strahlengänge vollständig öffnen, so daß eine binokulare oder monokulare Betrachtung der Sehzeichen mit dem linken oder rechten Auge möglich ist.

Bei einer selektiven Ansteuerung der LCD-Verschlüsse 1 kann das Muster, in dem der LCD-Verschluß den Strahlengang schließt, beispielsweise ein Zentrierkreuz sein. Natürlich sind aber auch andere Muster, wie Lochblenden oder dgl. möglich.

Auch können die LCD-Verschlüsse so angesteuert werden, daß sie eine Zwischenblende bilden.

In jedem Falle steuert die Steuereinheit jeden LCD-Verschluß 1 bzw. 1' derart an, daß er den jeweiligen Teilstrahlengang bzw. das Muster kontinuierlich öffnet bzw. schließt, so daß beispielsweise ein "weicher" Übergang von einem Modus zum anderen möglich ist.

## Patentansprüche

1. Vorrichtung zur Untersuchung des Sehvermögens, mit
- Einblick- bzw. Durchblicköffnungen (D),
- einem sich an die Einblick- bzw. Durchblicköffnungen anschließenden binokularen optischen Strahlengang, durch den eine untersuchte Person Sehzeichen oder dgl. betrachtet,
- einem in dem binokularen Strahlengang angeordneten optischen System (S bzw. L), und
- einem jedem Teilstrahlengang zugeordnetem Verschluß,
dadurch **gekennzeichnet**, daß der Verschluß ein LCD-Verschluß (1, 1', 1'') ist, der im Bereich des optischen Systems mit optischer Wirkung jedes Teilstrahlengangs vorgesehen ist, und daß eine Steuereinheit vorgesehen ist, die die LCD-Verschlüsse einzeln derart selektiv ansteuert, daß jeder LCD-Verschluß den zugeordneten Teilstrahlengang in bestimmter, vorgebbarer Weise in Form eines Musters abschwächt sowie den Teilstrahlengang und das Muster kontinuierlich öffnet bzw. schließt.

2. Vorrichtung nach Anspruch 1,
dadurch **gekennzeichnet**, daß das Muster, in dem der LCD-Verschluß den Strahlengang öffnet bzw. schließt, ein Zentrierkreuz oder eine Lochblende ist.

3. Vorrichtung nach Anspruch 1 oder 2,
dadurch **gekennzeichnet**, daß das Muster eine Zwischenblende bildet.

4. Vorrichtung nach einem der Ansprüche 1 bis 3,
bei der zur subjektiven Refraktionsbestimmung in jeden Teil-Strahlengang eine Reihe von Testlinsen in Richtung der optischen Achse beabstandet derart einbringbar sind, daß sie eine bestimmte optische Wirkung in dem jeweiligen Teil-Strahlengang erzeugen, dadurch **gekennzeichnet**, daß der jeweilige LCD-Verschluß vor oder hinter den Testlinsen angeordnet ist.

5. Vorrichtung nach einem der Ansprüche 1 bis 3,
bei der zur Durchführung eines Sehtests in jedem Strahlengang ein Linsensystem derart angeordnet ist, daß die untersuchte Person unter Fernsicht- oder Nahsicht-Bedingungen auf Sehzeichen oder dgl. blickt, dadurch **gekennzeichnet**, der jeweilige LCD-Verschluß in dem Linsensystem angeordnet ist.

## Claims

1. Device for examining visual acuity with
- openings for looking into or through (D),
- a binocular optical beam path following the openings for looking into or through, through which a person being examined observes visible symbols or the like,
- an optical system (S or L) located in the binocular beam path, and
- a closure associated with each partial beam path,
characterised in that the closure is an LCD closure (1, 1', 1'') which is provided in the region of the optical system with an optical effect on each partial beam path, and in that a control unit is provided, which selectively controls the LCD closures individually in such a way that each LCD closure attenuates the associated partial beam path in a specific predeterminable way in the form of a pattern, and continuously opens or closes the partial beam path and the pattern.

2. Device according to claim 1,
characterised in that the pattern in which the LCD closure opens or closes the beam path, is a centering cross or an apertured diaphragm.

3. Device according to claim 1 or 2,
characterized in that the pattern forms an intermediate diaphragm.

4. Device according to one of claims 1 to 3,
in which, in order to determine subjectively the refraction in each partial beam path, a series of test lenses may be brought in a spaced-apart manner in the direction of the optical axis in such a way that they generate a specific optical effect in the respective partial beam path, characterized in that the respective LCD closure is located in front of or behind the test lenses.

5. Device according to one of claims 1 to 3,
in which, in order to carry out a visual test, a lens system is located in each beam path in such a way that the person examined looks at visible symbols or the like under conditions of distance vision or of close vision, characterized in that the respective LCD closure is located in the lens system.

## Revendications

1. Dispositif d'examen de l'acuité visuelle, comportant
- des orifices d'observation intérieure ou traversante (D),
- un chemin optique binoculaire des rayons se raccordant aux orifices d'observation intérieure ou traversante par lequel la personne examinée observe des signaux optiques ou des objets similaires,
- un système optique (S ou L) disposé dans le chemin optique binoculaire, et
- un dispositif de fermeture affecté à chaque chemin partiel de rayons,
caractérisé en ce que le dispositif de fermeture est un dispositif de fermeture LCD (affichage à cristaux liquides) (1, 1', 1''), qui est prévu dans la zone du système optique, avec un effet optique sur chaque chemin partiel de rayons et en ce qu'un dispositif de commande est prévu, lequel commande sélectivement et individuellement les dispositifs de fermeture de façon telle que chaque fermeture LCD restreint ou affaiblit le chemin partiel de rayon(s) qui lui est affecté, d'une manière déterminée, susceptible d'être préalablement prescrite, sous forme d'un dessin ou d'un modèle, ainsi qu'elle ouvre, ou ferme, de façon continue le chemin partiel de rayons et le dessin ou modèle.

2. Dispositif selon la revendication 1,
caractérisé en ce que le dessin ou modèle sur lequel le dispositif de fermeture ouvre ou ferme le chemin de rayons est une croix de centrage ou un écran ou un diaphragme muni d'un trou.

3. Dispositif selon la revendication 1 ou 2,
caractérisé en ce que le dessin forme un diaphragme intermédiaire.

4. Dispositif selon l'une des revendications 1 à 3, dans lequel, en vue de la détermination subjective de la réfraction, il est possible de mettre en place dans chaque chemin de rayons partiel une série de lentilles d'essai à distance les unes des autres dans la direction de l'axe optique, de façon telle qu'elles produisent un effet optique déterminé dans le chemin partiel de rayons considéré, caractérisé en ce que le dispositif de fermeture LCD considéré est disposé en amont ou en aval des lentilles d'essai.

5. Dispositif selon l'une des revendications 1 à 3, dans lequel, pour l'exécution d'un essai de vue, un système de lentille est disposé dans chaque chemin de rayons, de façon telle que la personne examinée regarde le signe optique, ou l'objet similaire, dans des conditions de vue à grande distance, ou de vue à courte distance,
caractérisé en ce que chaque dispositif de fermeture LCD est disposé dans le système de lentilles.
